(19) Europäisches Patentamt / European Patent Office / Office européen des brevets

(11) **EP 3 691 798 B1**

(12) **EUROPEAN PATENT SPECIFICATION**

(45) Date of publication and mention of the grant of the patent:
**02.11.2022 Bulletin 2022/44**

(21) Application number: **18782141.8**

(22) Date of filing: **26.09.2018**

(51) International Patent Classification (IPC):
**B06B 1/02** (2006.01)     **A61K 41/00** (2020.01)
**A61K 9/50** (2006.01)

(52) Cooperative Patent Classification (CPC):
**B06B 1/0215; A61K 9/5094; A61K 41/0047;**
B06B 2201/76

(86) International application number:
**PCT/GB2018/052736**

(87) International publication number:
**WO 2019/069050 (11.04.2019 Gazette 2019/15)**

(54) **MAGNETO-ACOUSTIC DEVICE**

MAGNETO-AKUSTISCHE VORRICHTUNG

DISPOSITIF MAGNÉTO-ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.10.2017 GB 201716237**

(43) Date of publication of application:
**12.08.2020 Bulletin 2020/33**

(73) Proprietor: **Oxford University Innovation Limited Oxford, Oxfordshire OX2 0JB (GB)**

(72) Inventors:
• **BARNSLEY, Lester**
  **80804 Munich (DE)**
• **GRAY, Michael**
  **Oxford**
  **Oxfordshire OX4 1EA (GB)**
• **STRIDE, Eleanor**
  **Oxford**
  **Oxfordshire OX2 6UG (GB)**
• **PANKHURST, Quentin**
  **London**
  **Greater London W1T 4TP (GB)**

(74) Representative: **J A Kemp LLP**
**80 Turnmill Street**
**London EC1M 5QU (GB)**

(56) References cited:
**WO-A1-2007/028984     WO-A1-2012/063778**
**KR-A- 20130 125 054     US-A1- 2011 172 486**

**Description**

The present invention relates to a device for magnetic drug targeting. In particular the present invention relates to a magnetic-acoustic device (MAD) for controlling micro and nanoparticles both magnetically and acoustically.

Magnetic drug targeting (MDT) has recently received focus from researchers as a method for targeted drug delivery, due to its ability to localize and enhance the concentration of therapeutic agents in a specific, target region. Targeting of therapeutics that carry superparamagnetic nanoparticles and can be manipulated non-invasively by an externally applied field is seen as a promising means for improving the effectiveness of therapy and overcoming the risks and inefficiencies associated with systemic administration. However, there are a number of challenges to overcome before the technique can be considered clinically viable. In particular, while it is well recognized that the carrier formulation needs to be optimized for the application, it is increasingly apparent that the external magnet should be designed to generate a sufficient magnetic force over the target range to retain a useful proportion of carrier particles from the hydrodynamic flow of the circulatory system. Another problem with MDT is that the presence of strong magnetic forces can complicate imaging, making it difficult to gather reliable information regarding the effectiveness of a treatment protocol during therapy.

Ultrasound is a widely-used imaging modality that is fundamentally compatible with magnetic targeting, as acoustic and magnetic fields do not interact in biological systems. Microbubbles have been used clinically for decades as an ultrasound contrast agent due to their strong, non-linear response to acoustic fields. Recent work has focused on investigating the potential of microbubbles as vehicles for magnetic targeting by embedding superparamagnetic iron oxide nanoparticles (SPIONs) into their structure. Additionally, microbubbles can be formulated to carry drug molecules, and can enhance therapeutic outcomes due to microstreaming or cavitation. Ultrasound induced cavitation of drug-loaded microbubbles can also be used as an external trigger to disrupt the microbubble structure for controlled drug release.

In general, the external magnetic field is used to increase the local concentration of microbubbles in the diseased region (the target), either using an electromagnet or a permanent magnet array (i.e., magnetic drug targeting), and then a separate ultrasound transducer element/array applies ultrasonic excitation to the microbubbles to release the drug and/or force drug molecules to penetrate into the target. However, as the respective fields must be applied from sources outside the body, and commonly available field sources only have limited range, aligning both field sources to the same target is often a difficult geometric problem, which means that often one field source must be removed when the other is in operation, reducing the overall effectiveness of the treatment. US2011/172486 discloses methods of preparing and using magnetic microbubbles.

It is an aim of the present invention to at least partially address the above problems.

The invention provides a hand-held device for performing magnetic drug targeting on a patient, comprising: a magnetic element for providing a magnetic field configured to retain magnetic microbubbles within a target volume; an ultrasound element for providing an acoustic field configured to excite the magnetic microbubbles while the magnetic microbubbles are retained by the magnetic field within the target volume. Accordingly, the efficacy of treatments using the device may be increased compared to conventional methods because the microbubbles can be ruptured by the ultrasound without having to release the magnetic force applied to retain the microbubbles at the target.

The ultrasound element is mounted to the magnetic element so as to have a fixed spatial relationship to the magnetic element. Integrating the ultrasound and the magnetic element in this way allows the microbubbles to be more easily controlled simultaneously by the magnetic element and the ultrasound element.

The magnetic element may be configured such that the magnitude of a magnetic force exerted on microbubbles varies along an axis extending through the magnetic element and has a peak located a finite distance from the magnetic element on the axis. The ultrasound element may be configured such that an axis through the focal point of the acoustic field and the ultrasound element is co-aligned with the axis along which the peak magnetic force is located. This allows a user of a device to easily direct both the ultrasound and magnetic fields.

The ultrasound element may be mounted within a recess in the magnetic element. A first part of the magnetic element may comprise a channel therethrough for cooling the ultrasound element, the channel being in communication with the recess. This improves the performance of the ultrasound element and prevents damage to the ultrasound element.

A second part of the magnetic element may be detachably connected to the first part of the magnetic element and arranged behind the first part relative to the target volume. This makes the device easier to construct.

The invention will be described below in further detail by way of non-limiting examples, with reference to the accompanying drawings, which are briefly described below.

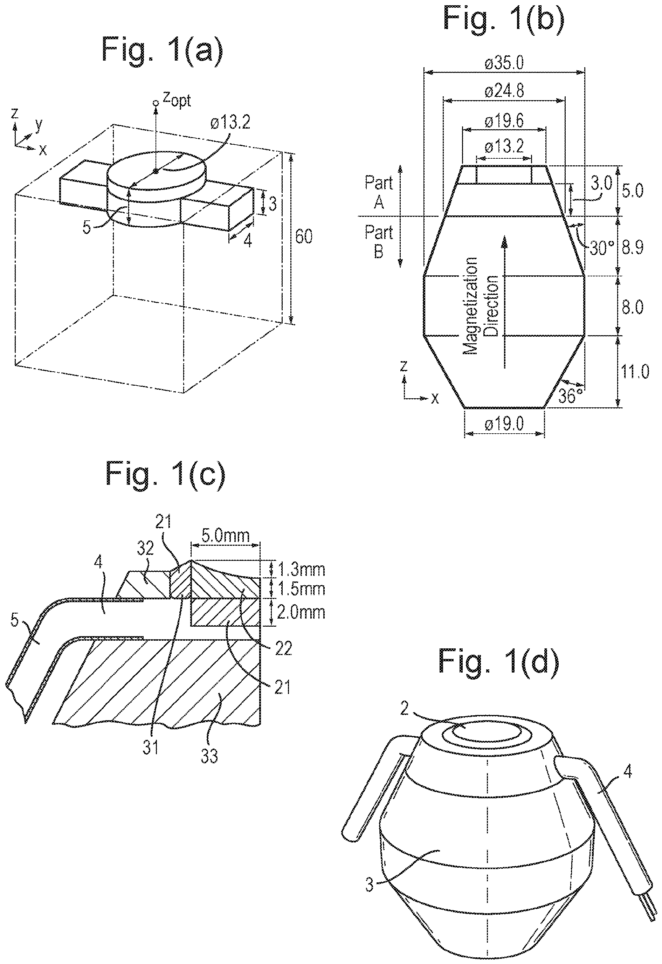

Figure 1: (a) Schematic of the optimization domain used to generate a design for a magnet with uniform magnetization, optimized to apply magnetic force to position of interest marked as $z_{opt}$. The lightly shaded surface shows the *x-y* plane, and the origin is referenced by a circle. The solid volume was excluded from the optimization to make space for an integrated ultrasound transducer and auxiliary components. (b) Cross-section in the x-z plane of the magnet design based magnetization directions to self-assemble in only one stable configuration. (c) Ultrasound element assembly showing piezoelectric disk and glass lens. (d) MAD assembly. All dimensions in mm.

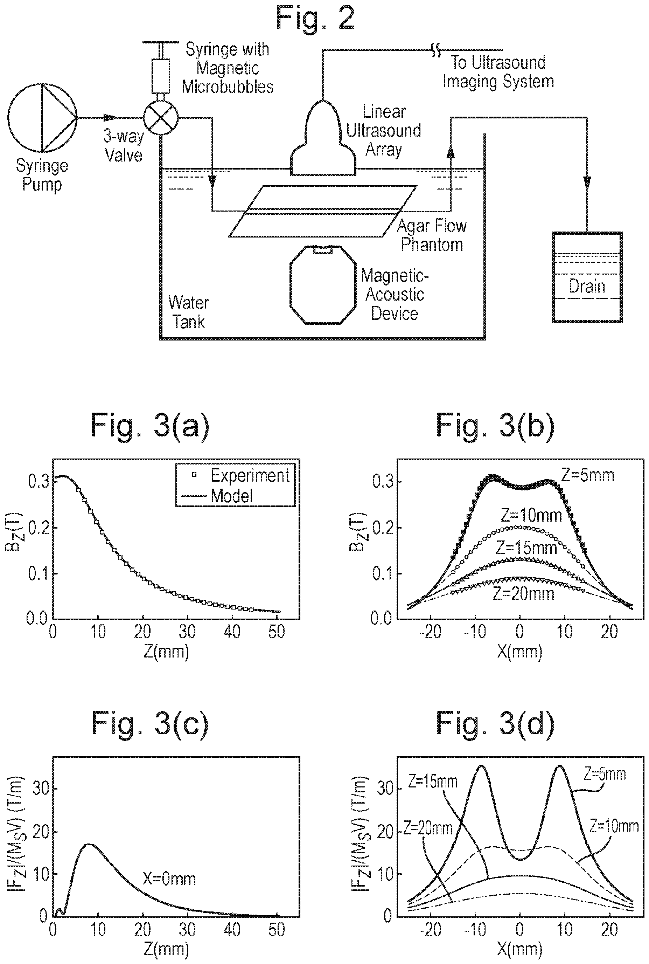

Figure 2: Schematic of set-up for ultrasound imaging experiments. A linear ultrasound array was used to monitor the accumulation of magnetically captured microbubbles. The MAD was coupled to an agar flow phantom, and microbubbles were injected into a steady flow created by a syringe pump.

Figure 3: Field profiles along the (a) z-axis and (b) x-axis at various depths, z away from the face of the magnet. The z-component of the field was measured using a Hall probe (symbols) and compared with simulation (lines). Predictions for the z-component of the normalized force are shown in (c) along the z-axis and (d) parallel to the x-axis at different z positions.

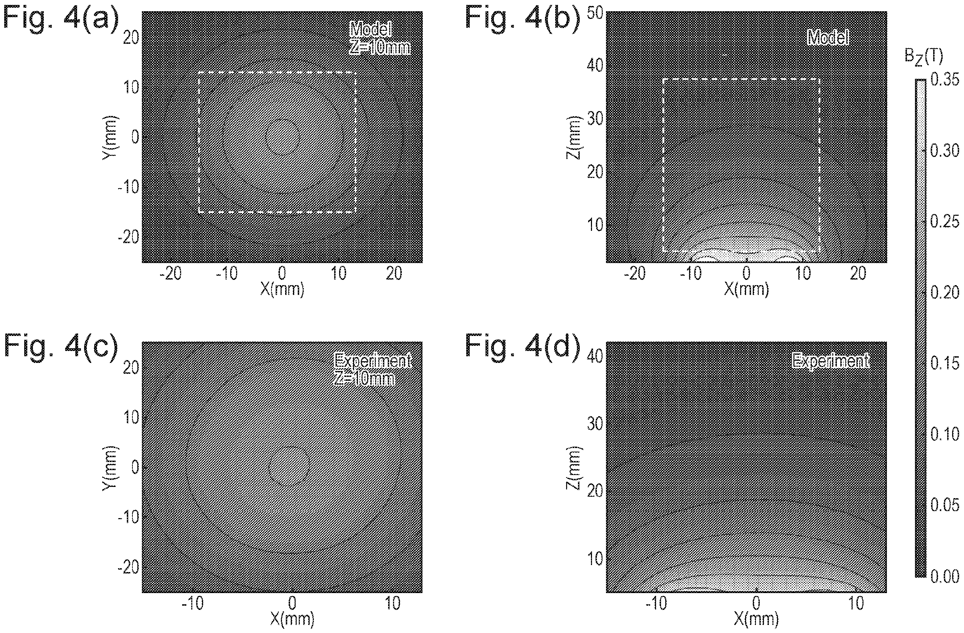

Figure 4: Maps of the z-component of B simulated in the (a) x-y plane at a range 10 mm above the surface of the MAD and (b) in the x-z plane. Hall probe measurements of subsets of the same planes are respectively shown in (c) and (d).

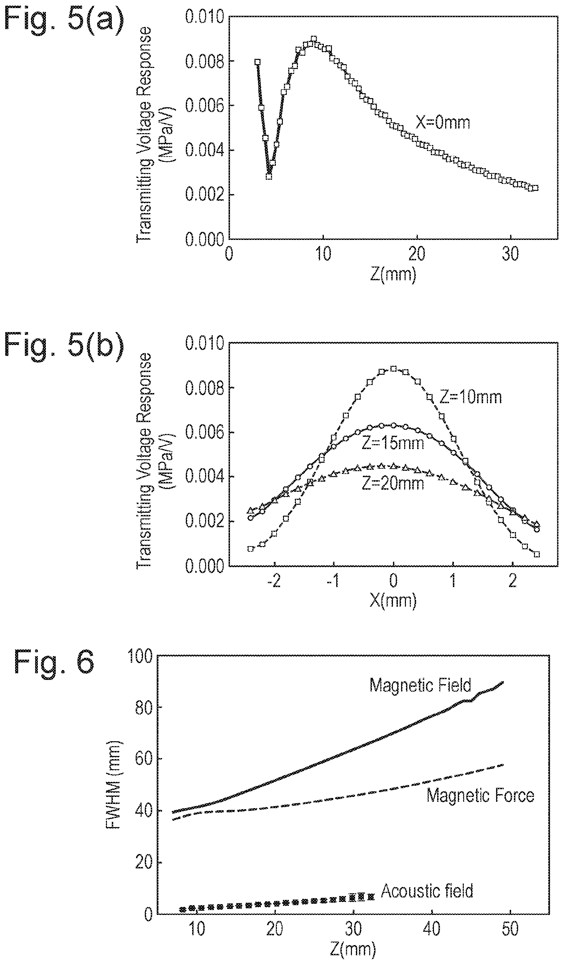

Figure 5: Transmitting voltage response profiles at 1.06 MHz on-axis (a) and radially (b) for three depths.

Figure 6: Full-width half-maximum of the experimental acoustic field, and the simulated magnitude of the magnetic field and force.

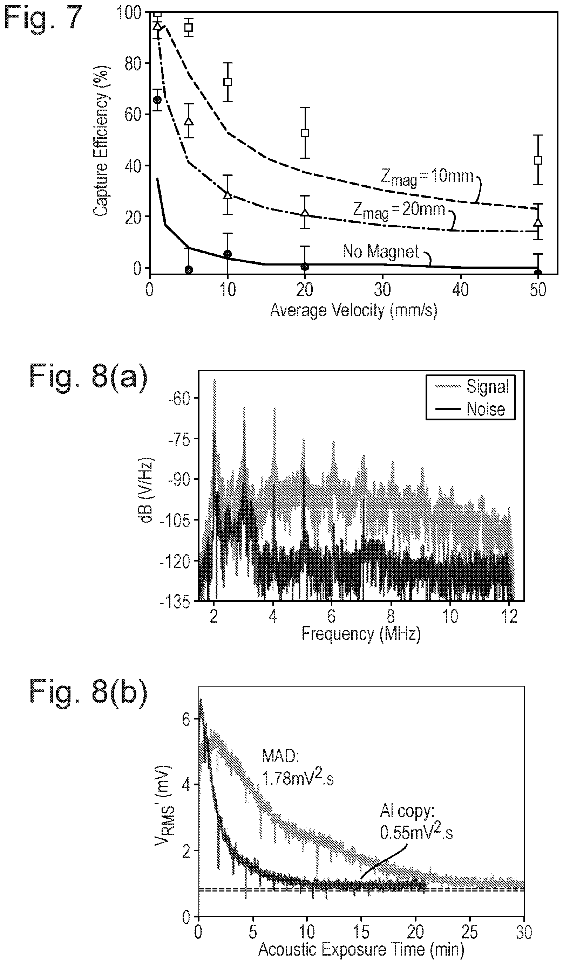

Figure 7: The capture efficiency measured by flowing microbeads in various fluid velocities with the channel set 10 and 20 mm from the MAD. The lines show predicted values for the capture efficiency using the model described above. The "no magnet" case is taken as the proportion of particles yet to reach the outlet after a simulation time of 2 minutes.

Figure 8: PCD data from MMB flow phantom experiments. (a) Spectral densities of a signal taken with the MAD activating retained MMBs (signal) and a channel flushed with water (noise). (b) RMS PCD voltage as a function of time after start of acoustic exposure. The horizontal dashed lines indicate $\pm 1$ standard deviation of the background noise. Cumulative PCD energy values were calculated over the measurement time and displayed with units in $mV^2.s$.

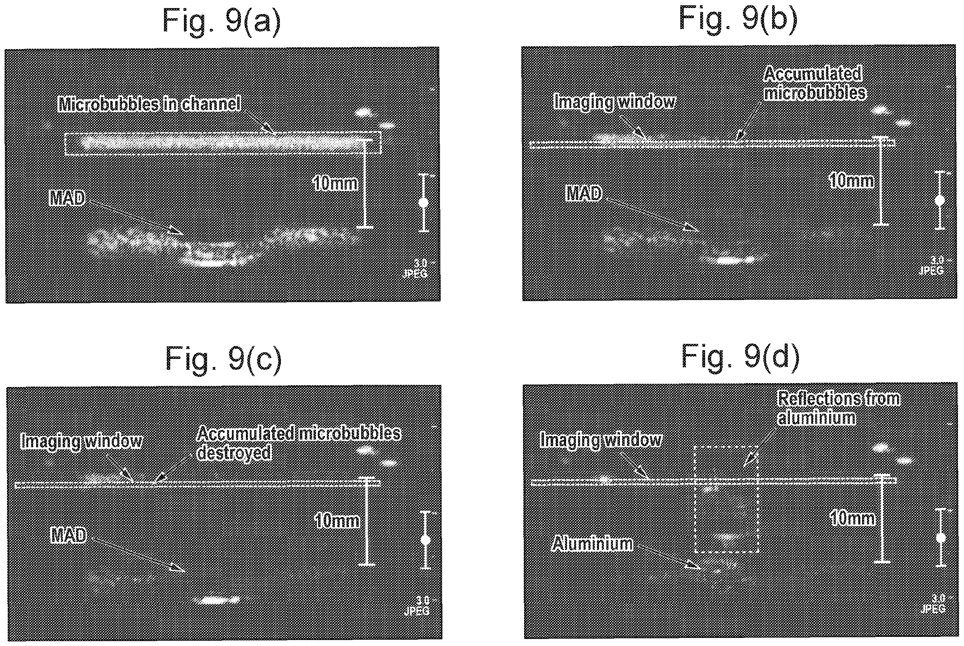

Figure 9: B-mode ultrasound images of magnetic microbubbles inside an agar flow phantom in range of the magnetic-acoustic device. (a) Microbubbles were injected into a steady flow inside the channel. (b) After the initial wave of the fastest microbubbles had passed, a captured bolus of accumulated microbubbles could be observed. The intensity profile inside the imaging window was analysed, (c) To verify that the accumulated particles were microbubbles, a momentary "flash" of high intensity ultrasound was applied, destroying the captured particles. Images in (a) - (c) are from the same video, while (d) is from a video recorded with an aluminium copy in place of the MAD. Elevated reflections in (d) were caused by a slight difference in B-mode probe angle for the MAD and aluminium experiments.

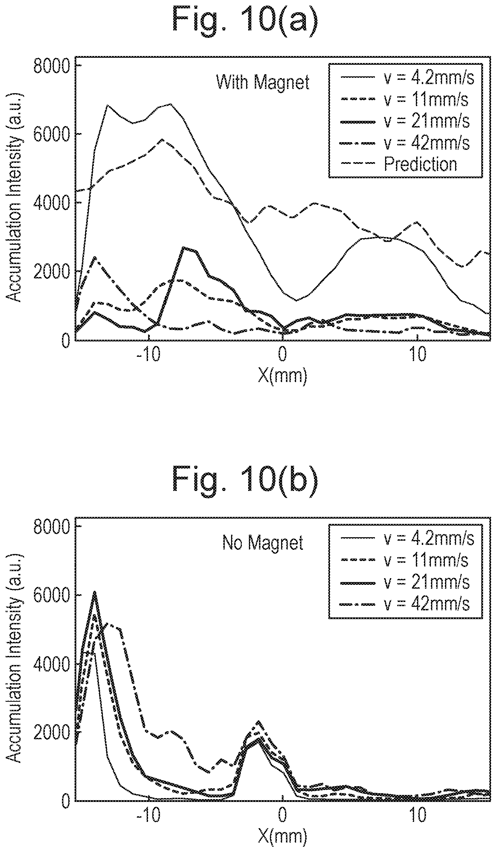

Figure 10: (a) Ultrasound intensity profiles along bottom of channel shortly after starting B-mode due to microbubbles captured by the MAD, with the flow velocity inside the channel varied between 4.2 and 42 mm/s. The dashed line is a prediction of the relative linear density of captured particles, calculated using the model described above. (b) Intensity profiles measured with the same conditions as (a), except with the MAD replaced with the aluminium copy.

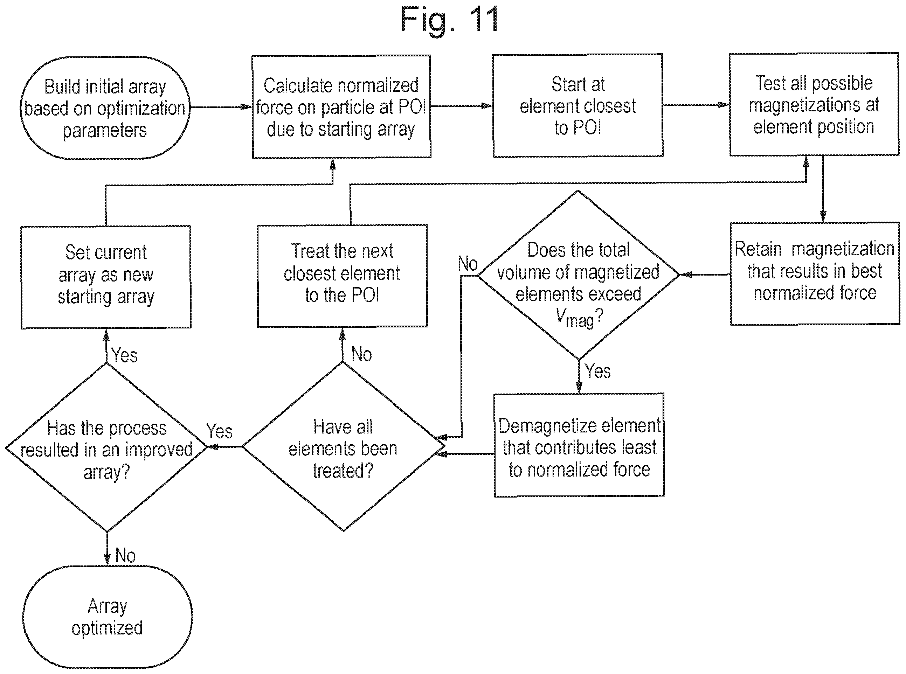

Figure 11: Flow diagram representing the routine for optimizing magnet arrays within an arbitrary parameter space.

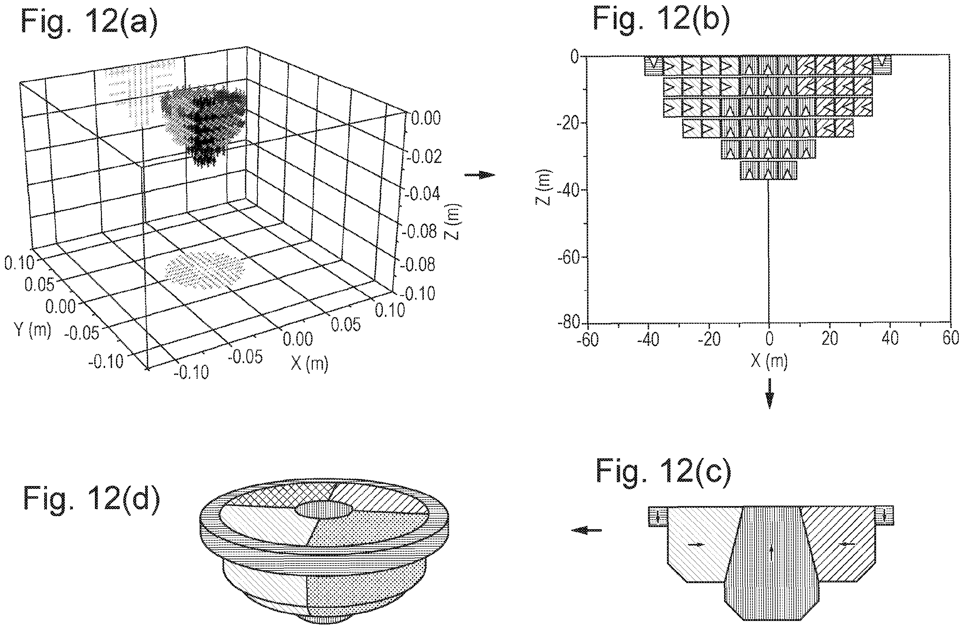

Figure 12: (a) The result of an optimization is given in terms of an arrangement of magnetization vectors which each represent the final orientation of an element in space. Vector arrows indicate the magnetization direction. Projections onto the x-y and x-z planes are displayed on the back-planes. (b) Where the output can be approximated by a cylindrically symmetrical arrangement, the optimized configuration is projected onto a 2D plane to generate a 2D vector map of a side cross-section through the middle of the array and (c) regions with the same magnetization are merged into individual shapes. (d) The resultant magnet arrangement can then be specified in terms of a series of cylindrically symmetrical segments with different dimensions.

Figure 13: (a) a further embodiment of the magnetic-acoustic device; (b) the magnetic-acoustic device within a holder.

According to an embodiment, an example of which is shown in Figure 1, there is provided a device 1 for magnetic drug targeting, comprising: a magnetic element 3 for providing a magnetic field configured to retain magnetic microbubbles within a target volume; an ultrasound element 2 for providing an acoustic field configured to excite the magnetic microbubbles while the magnetic microbubbles are retained by the magnetic field within the target volume. Accordingly, the efficacy of treatments using the device may be increased compared to conventional methods because the microbubbles can be ruptured by the ultrasound without having to release the magnetic force applied to retain the microbubbles at the target.

The ultrasound element 2 is mounted to the magnetic element 3 so as to have a fixed spatial relationship to the magnetic element 3. Accordingly, the device 1 according to the invention is easier to operate than conventional devices because it includes both ultrasound and magnetic elements integrated in a single device.

The magnetic element 3 may be configured such that the magnitude of the magnetic force exerted on microbubbles varies along an axis extending through the magnetic element (e.g. through one or more bodies of magnetic material forming the magnetic element 3) and has a peak located on the axis at a finite distance from the magnetic element 3. The magnetic element 3 is preferably configured such that the peak force along the axis is located within the target volume. A sharp rise and fall in the magnetic force along an axis results in improved retention of microbubbles in line with the axis. There may be a plurality of peaks and the magnetic element 3 may be configured such that any peak is located within the target volume. However, preferably the maximum peak is located within the target volume. The peak magnetic field generated may be from 0.1 T to 10 T. The peak magnetic field gradient generated may be from 1 T/m to 100 T/m.

A magnetic field providing a peak force may be provided by an annular portion of magnetic material forming the magnetic element 3. In this case, the axis on which the peak magnetic force is located coincides with a central axis of the annulus. Alternatively, or additionally, the magnetic material forming the magnetic element 3 may be tapered towards the location of the peak force. The taper in the magnetic material forming the magnetic element 3 may be a continuous taper, or the taper may be stepped, as shown in Fig. 12c. These structures compromise the magnetic force close to the magnetic element 3.

The magnetic material forming the magnetic element 3 may additionally be tapered in a direction away from the location of the peak force in a region of the magnetic material on an opposite side of the magnetic material to the other tapered region.

The body of magnetic material is preferably shaped so as to have a cylindrical portion, a tapered portion at one end of the cylindrical portion (facing the target) and, optionally a tapered portion at the other end of the cylindrical portion.

The magnetic element 3 may be formed from a plurality of bodies of magnetic material having different magnetisation directions (see Fig. 12c). This can provide better control over the magnetic force. For example, the magnetic element 3 may comprise a two different bodies of magnetic material respectively having magnetisation directions having a component in an opposite directions. A body of magnetic material having a magnetisation in one direction may be arranged to surround a body of magnetic material having an opposing magnetisation direction, the bodies being arranged in a part of the magnetic element 3 close to the location of peak force.

A first body of magnetic material is preferably shaped so as to have a cylindrical portion, a tapered portion at one end of the cylindrical portion (facing the target) and, optionally a tapered portion at the other end of the cylindrical portion. A second body of magnetic material, having an opposite magnetisation direction to the first body of the magnetic material, is preferably annular in shape and arranged to surround the first body such that a central axis of the annular shape and a central axis of the cylindrical shapes coincide.

Preferably the one or more bodies of magnetic material forming the magnetic element 3 have the same magnetisation direction. This simplifies the construction of the device because different parts of the device corresponding to different bodies of magnetic material do not repel each other.

Preferably, the one or more bodies of magnetic material forming the magnetic element 3 are arranged to have substantially cylindrical symmetry (e.g. with the exception of the channel 4 described below). In other words, the magnetic material forming the magnetic element 3 may have a circular cross section (a cross a longitudinal axis of the magnetic element 3). The axis of symmetry preferably coincides with the axis along which the peak force is located. Such an arrangement is shown in Figs 1c and Id.

The magnetic material forming the magnetic element 3 is preferably a permanently magnetic material, e.g. NdFeB. This type of magnetic material is suitably strong. The magnetic material may have a magnetization from 1.0 T to 1.5 T.

The magnetic element 3 may have a maximum width of from 2.5 cm to 15 cm (e.g. diameter in the case of an element having a circular cross-section). The magnetic element 3 may have a maximum length of from 2 cm to 10 cm. Such dimensions are suitable for holding the device in one hand. However, larger dimensions may be used for specific applications.

The location of the peak magnetic force along the axis and a focal point of the acoustic field may be substantially coincident. The magnetic element 3 and the ultrasound element 2 may be configured such that both location of the peak magnetic force along the axis and a focal point of the acoustic field are located with the target volume. This feature may be advantageous because such an arrangement ensures that the microbubbles are subject to the maximum acoustic excitation and maximum magnetic force at the same location. This may improve the efficiency of the device. This may also allow the size of the device to be minimised. The focal point of the acoustic field may be the focal point when the ultrasound is applied to tissue or in water, for example.

The target volume may be located between 1 mm and 150 mm from the tissue surface of a patient (e.g. external skin surface or internal oesophageal surface). Typically, the target volume is between 1 mm and 50 mm from the tissue surface. Accordingly, the location of the peak magnetic force and/or focal point of the acoustic field may be configured to be 1 mm and 50 mm from the surface of the device 1.

The acoustic field and the magnetic field may be co-aligned. This may be advantageous because such an arrangement maximises the effectiveness of both the acoustic and magnetic fields at the target volume. This may also allow the size of the device to be minimised. For example, the magnetic element 3 and the ultrasound element 2 may be configured such that the axis along which the peak magnetic force is located and an axis through the focal point of the acoustic field and the ultrasound element 2 may be substantially co-aligned. For a substantially cylindrically symmetric magnetic element 3 and ultrasound element 2 the co-aligned axes may be axes passing through the centre of the magnetic element 3 and ultrasound element 2 respectively. Such an arrangement is shown in Figs 1c and ld.

The magnetic element 3 may comprise a recess 31 for accommodating the ultrasound element 2. The ultrasound element 2 is mounted in the recess 31. Such an arrangement is shown in Figs 1c and ld. The recess 31 may be formed within in a surface of the magnetic element 3. Preferably, the recess 31 is formed in a surface of the magnetic element 3 facing the target volume.

In one embodiment, shown in Figs 1c and ld, a first part 32 of the magnetic element 3 may surround the ultrasound element 2. The first part 32 of the magnetic element 3 may be, for example, substantially annular in shape. The ultrasound element 2 may be, for example, substantially circular shape and arranged in a substantially circular recess 31 defined by the annular first part 32. The ultrasound element 2 and/or magnetic element 3 may be formed from multiple parts which are arranged in the above shapes. Alternatively, these shapes may be formed from a single part of the ultrasound element 2 or magnetic element 3.

In another embodiment, not shown, the ultrasound element 2 may surround the first part 32 of the magnetic element 3. The ultrasound element 2 may be substantially annular in shape, for example. The first part 32 of the magnetic element 3 may be substantially circular in shape, for example. The recess 31 may be substantially annular in shape to accommodate the ultrasound element 2. The ultrasound element 2 and/or magnetic element 3 may be formed from multiple parts which are arranged in the above shapes. Alternatively these shapes may be formed from a single part of the ultrasound element 2 or magnetic element 3.

In addition to the first part 32 of the magnetic element 3, the magnetic element may comprise a second part 33. The first part 32 of the magnetic element 3 may be arranged closer to the target volume relative to the second part 33. The second part 33 of the magnetic element 3 may be arranged adjacent and behind the first part 32 relative to the target volume. Such an arrangement is shown in Figs 1c and ld. The first part 32 may be tapered towards the target volume, as described above. The second part 33, may be tapered away from the target volume, as described above.

As shown in Fig 1c, a channel 4 may be provided in the magnetic element 3 adjacent the ultrasound element 2, for cooling cool the ultrasound element 2. Such an arrangement may be advantageous because it allows the performance of the ultrasound element 2 to be maximised and prevents damage to the ultrasound element 2 caused by overheating.

For example, the first part 32 of the magnetic element 3 may comprise a channel 4 therethrough. The channel 4 is preferably in communication with the recess 31. For example, the channel may pass through the recess 31. Such an arrangement is shown in Fig 1c. Alternatively, the second part 33 of the magnetic element 3 may comprise a channel 4 therethrough in communication with the recess 31 such that the top of the channel 4 connects with the bottom of the recess 31. Alternatively, the channel may be formed in both the first and second parts of the magnetic element 32, 33. The channel 4 channel preferably passes completely through the magnetic element 3 to provide separate input and outputs for a cooling fluid (such as air) to pass through the channel 4. The channel 4 may have a circular or rectangular cross-section, for example.

The channel 4 may additionally allow electric wiring to be connected to the ultrasound element 2 from outside the device e.g. for providing power to the ultrasound element 2 and/or controlling ultrasound element 2. Wiring and/or cooling fluid may be provided to the channel through one or more tubes 5 connected to the channel 4 through the openings of the channel 4 in the surface of the magnetic element 3.

Preferably, the second part 32 of the magnetic element 3 is detachably connected to the first part 32. The first and second parts 32, 33 are preferably formed from separate bodies of magnetic material having the same magnetisation direction when the parts 32, 33 are joined. Accordingly, the parts can be joined magnetically. Constructing the magnetic element in multiple parts makes the construction of the device 1 easier. For example, the recess 31 can be accessed from both sides of the first part 32 when the first part is separate from the second part 33 which makes mounting of the ultrasound element 2 easier. Further, the channel 4 can be formed in a surface face of the first part 32 or second part 33 to be joined with an adjacent surface of the other second part 33, 32. Therefore the channel 4 can be accessed easily when the first part 32 and the second part 33 are separated.

The ultrasound element 2 is preferably located in a surface of the device facing the target volume. Such an arrangement is shown in Figs 1c and ld. This arrangement may be advantageous because the ultrasound element 2 can be brought into contact with the patient to maximise the effectiveness of the acoustic field generated by the ultrasound element 2.

The ultrasound element 2 may comprise a piezo-electric transducer 21. This may be advantageous because this allows the ultrasound element 2 to be relatively compact in size. Other potential types of ultrasound elements may include capacitive micromachined ultrasound transducers (CMUTs) or an array of piezoelectric and/or CMUT elements. The ultrasound element 2 may generate ultrasound with a frequency of from 0.5 MHz to 10.0 MHz. The ultrasound element 2 may have a width of from 10 mm to 100 mm (e.g. diameter for an element having a circular cross-section).

The ultrasound element 2 may comprise a lens 22. The lens 22 may focus the ultrasound towards the target.

This may enhance the coupling of sound between the ultrasound element and the media being targeted (e.g. biological tissue). The lens 22 may be formed from glass, for example. The lens 22 may be concave. For example, the lens 22 may have a flat surface in contact with an ultrasound source, such as a piezo electric transducer 21, and an opposing concave surface facing away from the ultrasound source. Such an arrangement may be advantageous because it allows the acoustic field generated by the ultrasound element 2 to be focused at the target thus maximising the effectiveness of the acoustic field at the target.

The ultrasound element 2 may be connected to the rest of the device, e.g. magnetic element 3, by a flexible material 23. Such an arrangement is shown in Figs 1c and Id. The flexible material 23 may be an elastic material, for example. The flexible material 23 may comprise silicone. The use of a flexible material 23 allows the ultrasound element 2 to oscillate relative to the rest of the device, thus generating the acoustic field.

Example

The combined magnetic-acoustic device 1 (MAD) according to an embodiment of the invention intrinsically provides simultaneous co-alignment of two externally applied fields: a magnetic field and an acoustic ultrasound field.

*Magnetic Element*

The magnetic field was generated from a uniformly magnetized volume of magnetic material as the magnetic element 3. The shape of the magnet was determined using the optimization routine described below, in order to generate the optimal magnetic force at a position of interest (POI), $z_{opt}$, in this case 10 mm from the face of the device.

In summary, the optimization routine considers possible magnetic configurations of a three dimensional arrangement of elements positioned within an optimization domain, retaining the magnetic configurations that result in the maximal magnetic force at the position of interest. The total magnet volume was constrained to 20 cm$^3$. The optimization domain is shown in Figure 1(a) within a cubic frame, along with a teal volume that was excluded from the optimization to make space for the components to apply the acoustic field (i.e. ultrasound element 2). The excluded volume consisted of a cylinder to accommodate a cylindrical piezoelectric transducer 21 and a rectangular cross-section channel 4 embedded within the magnet volume, to provide space for airflow around and wiring to the ultrasound element 2.

The shape of the magnetic element 3 that resulted from the optimization routine is shown in Figure 1(b). A single magnetization direction was chosen (as opposed to a Halbach array with multiple magnetization directions (Halbach K. Design of permanent multipole magnets with oriented rare earth cobalt material. Nuclear Instruments and Methods. 1980; 169: 1 - 10.) to simplify the assembly process. The magnet was manufactured as a bespoke design consisting of two parts 32, 33 made from N52 grade NdFeB permanent magnet material (Bunting Magnetics Europe Ltd., Berkhamsted, UK) with parallel magnetization directions, so that they would only self-assemble in one stable configuration due to dipole interactions. The top part, first 32 encapsulates the excluded volume, and contains a cylindrical recess and a rectangular cross-section channel 4 along the diameter on the side opposite the face. An aluminium copy was constructed with identical dimensions to be used as a non-magnetic control device during testing.

*Optimisation Routine for the Magnetic Element*

A general expression for the magnetic force, **F,** on a single domain superparamagnetic particle with a moment of $\mu = \mathbf{M}(\mathbf{B})V$ is given by

$$\mathbf{F} = \nabla(\mu \cdot \mathbf{B}) = V\nabla(\mathbf{M} \cdot \mathbf{B}), \qquad\qquad (1)$$

where **M** is the magnetization of the particle, which depends on the field, $V$ is the volume of the particle and $\mathbf{B} = \mu_0\mathbf{H}$ is the magnetic flux density, proportional to the applied field, **H.** As the particle is superparamagnetic, it is assumed that **M** and **B** are parallel. The magnetization of a superparamagnetic particle can be described using a Langevin function $L(y) = \coth(y)-1/y$,

$$M(H) = M_s L\left(\frac{M_s V \mu_0 H}{k_B T}\right), \qquad\qquad (2)$$

here $M_s$ is the saturation magnetization of the particle, H is the applied field inside the particle and $k_B T$ is the product of the Boltzmann constant and the temperature.

The field emitted by an array consisting of an arbitrary configuration of magnetic elements was calculated by breaking the magnet into a 3-dimensional arrangement of evenly distributed point moments, following a method described previously (Stride E. et al Halbach arrays consisting of cubic elements optimised for high field gradients in magnetic drug targeting applications. Physics in Medicine and Biology. 2015; 60: 8303). Each moment emits a dipole field described by

$$B_i(r') = \frac{\mu_0}{4\pi}\left(\frac{3r'(\mu_i \cdot r')}{r'^5} - \frac{\mu_i}{r'^3}\right) \qquad (3)$$

where $\mu_i$ = Md$V$ is the point moment, **M** is the magnetization of the permanent magnet, d$V$ is the volume occupied by the point and **r'** is the position vector relative to the point moment. In the optimization routine, the normalized magnetic force due to the field emitted by an array of magnets on a superparamagnetic particle at a position of interest (POI) was calculated. The normalized magnetic force (or force per moment) is given by

$$\frac{\mathbf{F}}{M_s V} = \frac{M}{M_s}\nabla(B) \qquad (4)$$

and has units of T m$^{-1}$. When the particle is saturated ($M = M_s$), the normalized force is equivalent to the field gradient emitted by the array. The magnetic particle considered here (e.g. superparamagnetic particle) has the same saturation magnetization as $Fe_3O_4$ at room temperature ($M_s = 4.7 \times 10^5$ A m$^{-1}$) and a diameter of 10 nm.

The model was implemented using console applications written in the C# programming language (Microsoft Corporation, Redmond, WA, USA).

The optimization routine is able to generate designs of arbitrarily-shaped magnet arrays to deliver the maximal normalized force on a particle at the POI ($\mathbf{r}_{POI}$) given a series of design parameters, including the volume to be optimized, the nominal direction of normalized force ($\mathbf{F}_{nom}$), the volume of the magnet ($V_{mag}$), and the list of allowable magnetization directions contained within the array (Figure 11). An initial array is constructed to occupy the volume to be optimized consisting of both magnetized and non-magnetized elements, with magnetized elements occupying the positions closest to the POI. The total volume of the magnetized elements is limited to $V_{mag}$ at each step using a subroutine described below. The main routine then starts at the element closest to the POI and tests each allowable magnetization orientation, retaining the one that results in the best value of the optimized parameter, $\mathbf{F}(\mathbf{r}_{POI}) \cdot \mathbf{F}_{nom}/M_s V$ generated by the whole array at the POI. The process is then repeated for the next closest element until all elements in the array have been treated. At this point, convergence is tested by comparing the attained array to the configuration of the starting array. If the routine has changed the array and resulted in an improvement in the optimized parameter, the process is rerun using the attained array as the new starting array and again starting from the element closest to the POI until all elements have been treated. If the routine does not change the array after treating all elements and the optimized parameter cannot be improved, the array is considered optimized.

Whenever the combined volume of all elements with a non-zero magnetization exceeds the $V_{mag}$ parameter, a subroutine is performed in order to find and demagnetize the element that makes the least contribution to the normalized force. As the force depends on the gradient of the total field generated by the array at the POI, it cannot be assumed that this element is the element furthest from the POI. To find the element to demagnetize, each magnetized element is temporarily replaced by a non-magnetized element of the same volume and $\mathbf{F}(\mathbf{r}_{POI}) \cdot \mathbf{F}_{nom}/M_s V$ for the remaining array is recorded. The element that makes the least difference to the optimized parameter when replaced by a non-magnetic element is demagnetized.

An example magnet output from the optimization routine is shown in Figure 12. Space has not been allowed for an integrated ultrasound element 2 in this example.

*Ultrasound Element*

The ultrasound element 2 of this embodiment comprises a 10 mm diameter piezoelectric disk with 1 MHz resonant frequency and wraparound electrodes (e.g. from Noliac, Kvistgaard, Denmark). This was chosen on the basis of predicted acoustic field shape and estimated component cost. The 1 MHz operating frequency was chosen as a compromise between the modest range of attenuation values in biological soft tissues and the ability to produce suitable pressure amplitudes with a compact element (Duck F. Physical Properties of Tissue: A Comprehensive Reference Book. Academic Press. 1990). Acoustic field focusing was provided by a planoconcave glass lens 22 (GalvOptics, Essex, UK) with 10.3 mm radius of curvature. A BK-7 glass formulation was chosen to enhance acoustic impedance matching between the piezoceramic and the external acoustic environment (water or soft biological tissue). The lens 22 was fixed to one side of the piezoelectric disk using an epoxy (Araldite Ultra, Huntsman Advanced Materials, Everberg, UK) that was degassed for one minute after mixing.

The ultrasound element 2 provides a focused acoustic field that is spatially overlapped with the magnetic field peak with sufficient amplitude to cause inertial cavitation of candidate microbubble formulations. The ultrasound element 2 is sufficiently compact so that the excluded magnet volume (and corresponding compromise to the magnetic field) can be minimized.

Assembly of the MAD 1 comprises passive mating of the two magnet components (with care taken not to damage the nickel coating). Next, the acoustic element is centred 1.4 mm above the bottom of the excluded magnet volume using non-ferrous spacer rods, after which the perimeter gap between the acoustic element and magnet is sealed using silicone (Loctite SI 4145, Henkel Ltd., Hemel Hempstead, UK). Two additional applications of sealant are applied after the first has dried and the spacer rods are removed. To complete the assembly (Figure 1(d)), the rectangular openings in the magnet are fitted with flexible tubing to allow airflow around the acoustic element and to provide a waterproof path for the element drive wires. A final application of silicone is used to seal the tubing entry points and the two magnet sections.

N52 grade NdFeB was chosen for the magnet material due to it having one of the highest magnetization values of commercial NdFeB grades ($1.02 \times 10^6$ A/m), and a temperature rating of about 80°C (although flux loss can occur even at lower temperatures). Heat transfer from the active transducer 21 to the magnet material can be minimized by using a glue with low thermal conductivity to affix the transducer 21. The channel 4 to accommodate electrical wiring for the transducer 21 also serves an additional purpose, allowing ventilation for air-cooling during operation. Thermal testing performed using a series of fine needle thermocouples (Hypo 33-1-T, Omega, Stamford, CT, USA) to probe different positions on the MAD 1 during operation of the transducer 21 (1 MHz, 3000 cycle tone pulses with 75 V amplitude drive voltage and 30% duty cycle) showed a temperature rise of just 1.3°C over a 20 minute drive period. Relatively small values were chosen for $z_{opt}$, the optimization distance and $V_{mag}$, the magnet volume but, in principle, a larger device can be optimized for larger length scales. For example: $z_{opt}$ of from 5mm to 50mm and $V_{mag}$ of from 10 cm$^3$ to 1000cm$^3$.

## Experimentation

### Calibration of applied fields

Measurements of the vector magnetic field emitted by the magnet were performed using a three-axis Hall probe connected to a Model 460 3-Channel Gaussmeter (Lake Shore Cryotronics, Inc., OH, USA). The Hall probe was mounted on a set of three MTS Series Motorized Translation Stages (Thorlabs, Inc., NJ, USA) with travel ranges of 50 mm, configured to give controllable translation in each of three orthogonal directions.

Acoustic field profiles were measured with a needle hydrophone (200 $\mu$m needle, Precision Acoustics, Dorchester, UK) while the MAD 1 front face was submerged in a tank filled with filtered and degassed water. The ultrasound element was driven with a three cycle, 1 MHz tone burst from a waveform generator (33250, Agilent Technologies, Cheshire, UK) and amplified with a nominal gain of 55 dB (1040L, E&I Ltd., Rochester, NY, USA). Automated scan control software (UMS2, Precision Acoustics, Dorchester, UK) incrementally translated the hydrophone beneath the stationary MAD 1 and transferred its response signals from an oscilloscope (Waverunner 64Xi, Teledyne LeCroy, Geneva, Switzerland) to computer disk for analysis. Drive voltage (PP007-WR, LeCroy) and current (4100, Pearson Electronics, Palo Alto, CA, USA) probes were monitored to ensure proper system operation and allow subsequent calculation of electrical impedance. Calibration data sets were processed in MATLAB (The MathWorks Inc., Natick, MA, USA) using the following steps: i) application of a high pass filter to remove any DC offset in the data traces, ii) calculation of hydrophone $A(f,x,y,z)$ and drive voltage $V(f)$ Fourier transforms, and iii) calculation of the transmitting voltage response (TVR) at each frequency and scan grid point $(x,y,z)$: $TVR(f,x,y,z) = A(f,x,y,z)/(V(f)S(f))$ where $S(f)$ is the hydrophone sensitivity. Water temperature was monitored with a glass thermometer, with values used to estimate sound speed for use in estimating hydrophone position along the MAD 1 symmetry axis.

### Magnetic microbead retention experiments

Magnetic retention experiments were performed to demonstrate the effectiveness of the MAD 1 for retaining magnetic carriers against flow. Polystyrene magnetic microbead particles ($2.0$-$2.9 \times 10^{-6}$ m, Spherotech, Inc., Lake Forest, IL, USA) were used as model magnetic carriers, due to their relatively good monodispersity. The magnetic behaviour of the microbeads was characterized using a MPMS superconducting quantum interference device (SQUID) magnetometer (Quantum Design, Inc., San Diego, CA, USA) and exhibiting an effective, superparamagnetic cluster size of 8.6 nm and a 16.2% weight loading of iron oxide in polystyrene (Stride E. *et al* Understanding the dynamics of superparamagnetic particles under the influence of high field gradient arrays. Physics in Medicine and Biology. 2017; 62: 2333). The microbeads were diluted to a concentration of $4 \times 10^6$ mL$^{-1}$ and conveyed into a straight, cylindrical channel (1.2 mm diameter) embedded in a flow phantom using a syringe pump. The phantom consisted of a degassed mixture of 2.5% agar (UltraPure Agarose 1000, Life Technologies, Paisley, UK) and filtered water poured into a thin rectangular mold bounded by 0.015 mm thick mylar sheets (PMX980, HiFi, Hertfordshire, UK) to allow uninhibited acoustic transmission. The phantom frame, fasteners, and flow channel conduits were all made of non-ferrous polymer materials to avoid extraneous stray magnetic fields during retention tests. The MAD 1 was affixed to the outside of the phantom frame using a 3-D printed guiding ring, so that the relative position of the MAD 1 to the flow phantom could be reproducibly set between experimental runs. The magnet was set at either 10 or 20 mm away from the flow phantom, and the average fluid velocity in the flow channel was varied between 1 and 50 mm/s.

The capture efficiency was determined by comparing the concentration of microbeads before (initial) and after (final) the flow phantom. To measure the concentration, a series of images were obtained of microbeads using a 40× objective lens on a Leica DM500 optical microscope (Larch House, Milton Keynes, UK), and analyzed with a custom image processing routine based on the NumPy package for Python 3.5. The capture efficiency was calculated as:

$$C.E. = (C_i - C_f)/C_i \times 100\%.$$

The experiments were repeated using the non-magnetic aluminium copy of the MAD 1.

Predictions about the capture efficiency were made using a numerical model for particle trajectories (Stride E. et al, Understanding the dynamics of superparamagnetic particles under the influence of high field gradient arrays. Physics in Medicine and Biology. 2017; 62: 2333). In summary, simulations were performed of an ensemble of particles with the same magnetic properties as the microbeads, which were distributed evenly at the inlet of a channel carrying laminar flow. A force balance was used to solve the trajectories and calculate the proportion of particles that were captured by the magnet and the proportion that reached the outlet. Parameters were input to match the experimental conditions and the simulations were run until all particles reached their final position. The simulations were repeated without an external magnetic force over 2 minutes of simulation time only (as all magnet simulations had all particles reach their final positions within 2 minutes of simulation time).

*Magnetic microbubble acoustic intensity experiments*

Magnetic microbubbles were prepared by following an adapted method from Stride *et al.* (Stride E, et al. Enhancement of Microbubble Mediated Gene Delivery by Simultaneous Exposure to Ultrasonic and Magnetic Fields. Ultrasound in Medicine & Biology. 2009; 35: 861 - 868) as described below:

1,2-Distearoyl-sn-Glycero-3-Phosphocholine (DSPC) was purchased from Avanti Polar Lipids, Inc. (Alabaster, AL, USA). Polyoxyethylene (40) stearate (PEG40S), chloroform, Dulbecco's phosphate-buffered saline were purchased from Sigma-Aldrich Ltd. (Gillingham, Dorset, UK). Isoparaffin coated magnetic nanoparticles (10 nm diameter) were purchased from Liquids Research (Bangor, UK). Sulphur hexafluoride ($SF_6$) was purchased from The BOC Group (Guilford, Surrey, UK).

A mixture of DSPC:PEG40S in chloroform (9:1 molar ratio) was prepared by adding 621 μL of DSPC (25 mg/mL) and 447 μL of PEG40S (10 mg/mL) into a glass vial. The sample was covered with pierced parafilm and heated to 50°C overnight to evaporate the solvent.

After complete solvent evaporation, the dried lipid film was suspended in 5 mL of PBS for 1 h at 75°C under constant magnetic stirring. The stir bar was removed from the sample and the solution was sonicated using a XL2000 ultrasonic cell disruptor from Misonix, Inc. (Farmingdale, NY, USA). The sonicator was used at power setting 4 (8 $W_{RMS}$ output power) for 15 seconds with a 3-mm diameter tip, operating at 22.5 kHz, with the probe tip held within the solution. This was immediately followed by sonication at the gas-water interface with the probe tip touching the liquid surface, under positive pressure of $SF_6$ and at power setting 19 (38 $W_{RMS}$) for 10 seconds. 15 μL of isoparaffin coated iron oxide nanoparticles (10 nm diameter) was then added to the mixture and the vial was gently swirled for 10 seconds. The solution was again sonicated with the probe tip held within the liquid at power setting 4 for 15 seconds, followed by cooling of the sample in a 5°C fridge for 15 minutes. Then, the solution was again sonicated at the gas-water interface, under positive pressure of $SF_6$ at power setting 19 (38 $W_{RMS}$) for 10 seconds. Finally, the magnetic microbubble solution was capped and placed on ice for 10 minutes before further analysis.

Microbubbles were observed using a Leica DM500 optical microscope (Larch House, Milton Keynes, UK) with a 40× objective lens, and a haemocytometer from Hausser Scientific (Horsham, PA, USA). Microbubble concentration and size analysis was completed using a purposely-written image analysis software in MATLAB (Sennoga C A, et al. On Sizing and Counting of Microbubbles Using Optical Microscopy. Ultrasound in Medicine & Biology. 2010; 36: 2093 - 2096). On average (n = 5), each batch produced $(4.4 \pm 0.6) \times 10^8$ magnetic microbubbles/mL of solution of size $2.6 \pm 0.25$ μm.

In order to demonstrate that the MAD 1 could capture acoustically responsive magnetic carriers, microbubbles were diluted to 1/10 of the batch concentration and injected into a steady laminar fluid flow, established inside the agar flow phantom described above, using a syringe pump (Figure 2). The MAD 1 was fixed to the phantom holder at a distance 10 mm from the channel, as described above, and the average fluid flow velocity was varied between 4 and 42 mm/s. After waiting for 4 minutes (which, according to simulations, was sufficiently long for a captured bolus of magnetic microbubbles to form inside the channel near the magnet), the channel was imaged using a commercially available ultrasound system (iU22, Philips, Bothell, WA, USA) with a linear array (L12-5, Philips) angled approximately 40 degrees off the MAD 1 symmetry axis. Videos consisting of B-mode images were recorded for 1 minute at a frame rate of 13 frames/s.

An ultrasound drive level corresponding to a mechanical index (MI) value of 0.15 was used to image the accumulated bolus, but as the microbubbles were extremely acoustically responsive and not stable, the imaging system-generated pressure was already sufficient to destroy microbubbles. In order to determine the accumulated intensity from captured microbubbles, a series of frames in a 5 second window were selected for processing after any particles in flow had cleared, but before the intensity from captured microbubbles had decayed too much. These images were analyzed using a custom image processing routine based on the NumPy package for Python 3.5. The bottom of the channel in the images was windowed, and the position dependent intensity, $I(x)$ was determined by taking a weighted local regression of the total intensity in the part of the window between $x \pm \frac{1}{2}\mathrm{d}x$, which was then averaged for all selected images from the same video. All experimental runs were repeated with non-magnetic control device.

Experimental position dependent intensities were compared with numerical predictions for the accumulation distribution, which were calculated using a known model (Stride E. et al Understanding the dynamics of superparamagnetic particles under the influence of high field gradient arrays. Physics in Medicine and Biology. 2017; 62: 2333). The accumulation distribution was taken as the relative proportion of captured particles with simulated final positions ranging between $x \pm \frac{1}{2}\mathrm{d}x$.

The combined magnetic retention and acoustic activation capabilities of the MAD 1 were demonstrated by monitoring acoustic emissions from the flow channel while driving the ultrasonic element. The drive chain was the same as described in section 2.2, but the drive signal was lengthened to 100 cycles, and the pulse repetition rate slowed to 1.0s. The drive amplitude was set so that the peak rarefactional pressure at the center of the channel would be 0.50 MPa, based on the results of free field calibrations described in section 2.2. Ultrasonic emissions from the channel were observed using a spherically focused single element transducer 21 (7.5 MHz center frequency, 0.5" dia., 2.95" focus, Olympus NDT, Essex, UK) operating as a passive cavitation detector (PCD). Signals from the PCD were preamplified (SR445A, SRS, Sunnyvale, CA, USA), digitized (Handyscope HS3, TiePie Engineering, Sneek, Netherlands) upon triggering from the waveform generator, and streamed to a computer disk.

Prior to conducting cavitation monitoring experiments, alignment of the PCD with the section of channel directly in front of the MAD 1 was achieved by temporarily introducing an air pocket into the channel. The PCD was then connected to a pulser (5072PR, Olympus NDT), and its position adjusted to maximize the scattered signal amplitude within the expected propagation time window. For all experiments, the PCD was angled approximately 40 degrees above the (horizontal) beam axis of the MAD 1 element in order to minimize mutual scattering.

*Calibration Results*

It is well understood that the field and force profiles emitted by a magnetized volume depend on its shape. Hall probe measurements of the z-component of the external field, $B_z$ generated by the MAD 1 are shown in Figure 3(a) and (b), and showed good agreement with model predictions for its shape, particularly along the z-axis. Predictions for the z-component of the normalized force are shown in Figure 3(c) and (d). Typically, the force from a solid magnetic volume falls off almost exponentially with distance, but the recess in the front face of the magnet compromises the magnetic force at short range, and even produces a small push force within 2 mm of the magnet. The normalized force at the position of interest, $z_{opt}$, is 15.8 T/m, which compares well with the force expected from a magnet optimized for the same parameters without the excluded volume (about 18 T/m).

The compromise in performance at short range can be understood by examining the profiles in Figure 3(d). At z = 5 mm, the MAD 1 emits strong forces at the edges of the device and a relatively weaker central force. This is the type of force profile that typically results in more particles accumulating closer to the edge of the magnet, rather than above the centre, resulting in an inefficient accumulation distribution if the target is aligned co-axially with the MAD 1. Simulation results suggest that force profiles that rapidly vary and peak in a confined spatial region lead to more efficient accumulation of carriers to a co-axially aligned target. The MAD 1 emits this type of force profile beyond z = 15 mm, but at this range, the full-width half-maximum (FWHM) of the profile is already about 40 mm.

Figure 4 shows that Hall probe measurements of the field emitted by the MAD 1 agreed with simulations for the same planes. At a range of 10 mm from the surface of the array, simulations predicted a field of 0.203 T at the centre of the x-y plane (Figure 4(a)), compared with a measured field of 0.201 T (Figure 4(c)).

Figure 5 shows the measured acoustic field profiles for the MAD 1 ultrasound element at a frequency of 1.06 MHz, which was found to have the highest TVR in the 0.8 - 1.2 MHz data analysis band. The location of the focus was as designed (10 mm), with a gradual attenuation and broadening of the beam pattern with increasing post-focal depth. Calibration of the non-magnetic (aluminium body) device showed essentially identical frequency trends and field profiles to those shown in Figure 5, but with a modest global amplitude offset. This information was used to set drive voltage levels in subsequent retention and activation experiments, so that the output pressures would be the same for both devices.

The FWHM for each of the applied fields was determined from profiles parallel to the x-axis at different positions for z (Figure 6). It is noted that the acoustic field has a much more narrow profile than either the magnetic field or force, demonstrating that the MAD 1 typically activates a small portion of accumulated particles during application of the acoustic field.

*Magnetic capture efficiencies*

The performance of the MAD 1 to magnetically target microscopic carriers was characterized by measuring the proportion of magnetic microbeads that were captured inside a flow phantom at different distances from the magnet, and at a range of flow velocities (Figure 7). The results were compared with predictions made using the simulations described above, which were performed using particle parameters to match the magnetic properties measured for the microbeads. A slightly higher capture efficiency than predicted was observed for most conditions, which was most likely due to inter-particle interactions between the magnetized beads (interactions were ignored in the simulations for simplicity). Any offset in the magnet position with respect to the channel would also contribute to the discrepancy. However, both the measured and simulated capture efficiency values demonstrated that the MAD 1 was capable of capturing more than 10% of the injected particles for all flow velocities tested.

In the "no magnet" case for low velocities a relatively high "capture efficiency" (or, more accurately, a high proportion of unaccounted particles, as there was no external force to capture microbeads) was observed, as sampling was performed approximately 1 minute after injecting the particles. Simulations suggested, at these flow velocities, this was insufficient time for the concentration to equilibrate at the outlet of the phantom.

*Cavitation activity of captured Magnetic Microbubbles*

Figure 8 shows examples of PCD responses during the magnetic microbubble (MMB) retention and activation experiments. The average fluid velocity in the channel was 4.2 mm/s. In the presence of MMBs, the PCD frequency spectrum elevates above the MMB-free background measurement in both tonal and broadband levels (Figure 8(a)), indicating a mix of bubble behaviours (including inertial cavitation) for the incident field level used. The lack of ultraharmonics (half-integer harmonics of the 1.06 MHz drive frequency) suggests the absence of stable bubbles. Although the results in Figure 8(a) are for single acquisitions, they are representative of the ensemble of collected data. The temporal histories of PCD signals recorded with the magnetic and non-magnetic devices installed are shown in Figure 8(b). After exhibiting similar initial levels, the signals obtained with magnetic (MAD 1) and non-magnetic (Al copy) devices strongly diverge, with the MAD 1 significantly extending the time over which MMB responses are observable. The amount of time that the magnetically retained MMB response took to decay to half of its peak value (relative to the noise floor) was $322 \pm 52$ s, compared with $74 \pm 13$ s using the non-magnetic device, meaning that use of the MAD 1 effectively increased the sustained time of cavitation activity by a factor of 4.3. The cumulative signal energies (displayed in Figure 8(b) with units in $mV^2$.s) were calculated over a time span for which the root mean square (RMS) PCD signals are more than twice that of the background. Magnetic retention enhanced MMB response energy by a factor of 3.3.

*Ultrasound imaging of captured MMBs*

In order to demonstrate that the MAD 1 could capture and accumulate carriers that are responsive to both acoustic and magnetic stimulation, a B-mode ultrasound imager was used to examine microbubbles injected into an agar flow phantom coupled with the magnet. After waiting for set amount of time, a lingering intensity could be observed along the bottom of the channel (Figure 9(b)). As the microbubble formulation was particularly unstable, a "flash" of high intensity ultrasound could be used to destroy any remaining microbubbles via inertial cavitation. Figure 9(c) shows that, after flashing the channel, the intensity along the bottom of the channel disappeared, however, magnetic particles could still be seen in the vicinity of the magnet by visual inspection of the flow phantom as a build-up of brown discolouration. This verified that the intensity that persisted along the bottom of the channel was due to captured microbubbles.

Figure 10 shows intensity profiles from accumulated microbubbles along the bottom of the channel, which was determined by averaging a number of frames in a short timespan from each video, as described in section 2.4. The profiles recorded with the MAD 1 in a position 10 mm from the flow channel were compared with predictions for the relative accumulation distribution of captured particles made using the model described above. The model predicted that the greatest accumulation of particles would be observed in a region approximately 8 mm upstream from the centre of the magnet, and qualitatively comparable behaviour was seen for the intensity profiles, except at the highest flow velocity. The intensity was also analysed for videos recorded with no magnet (Figure 10(b)), which exhibited two peaks in all profiles. The upstream peak was due to fresh microbubbles flowing into the imaged area prior to being destroyed, while the central peak was seemingly unrelated to the presence of microbubbles, and instead was due to a persistent reflection from the aluminium body in the centre of the channel, as seen in Figure 9(d).

The magnetic element can be manufactured from relatively inexpensive and easy-to-assemble permanent magnet components. Using a grade of NdFeB with a high remanent magnetization has a number of advantages; as the magnetic energy is stored internally, no external power supply is required, meaning the device can be small and light-weight and slight (air) cooling is only required to keep the magnet well below the graded temperature during operation of the ultrasound transducer 21. Ventilation can be built into the device to dissipate heat generated by the transducer 21 away from the bulk of the magnetic material, and only mild heating was observed during testing.

In a further embodiment of the device shown in Fig. 13A, the device further comprises a coupling member 24, in order to provide an interface between the ultrasound element 2 and the target site of interest (e.g. skin of a patient). Similarly to previous embodiments, the magnetic element comprises N52 grade NdFeB permanent magnet material whose geometry is optimized to have a maximum magnetic field of around 0.2 T at a distance of 10 mm from the body's leading edge. An integral ultrasound element with the same focal distance provides a pressure field that spatially overlapped with the magnetic field peak, with sufficient amplitude to cause inertial cavitation of candidate microbubble formulations. Channels in the magnet body allowed airflow for passive cooling around the acoustic element, as well as a path for the element drive cable.

The coupling member 24 is located at and extends from the surface of the ultrasound element 2. The coupling member may be substantially conical in shape, tapering further from the ultrasound element 2. The coupling member 24 may have substantially cylindrical symmetry about an axis through the centre of the ultrasound element 2. In an example the coupling member was formed from paraffin wax (FullMoons Cauldron, Berkshire, UK) and secured with ultrasound gel (Anagel AW, Ana Wiz Ltd., Surrey, UK) as shown in Fig.13. The coupling member material was chosen for its ease of casting and minimal transmission loss in the 1 MHz frequency range as determined by conventional through- transmission measurements.

As shown in Fig 13B, the device may further be housed within a holder 5. The holder 5 may comprise a recess for accommodating the device. The holder may comprise handles 51 for the user to hold when operating the device. Two handles 51 may be provided, one on either side (e.g. opposing sides) of the holder 5.

**Claims**

1. A device for performing magnetic drug targeting on a patient, comprising:

   a magnetic element (3) configured to provide a magnetic field configured to retain magnetic microbubbles within a target volume; and
   an ultrasound element (2) configured to provide an acoustic field configured to excite the magnetic microbubbles while the magnetic microbubbles are retained by the magnetic field within the target volume, **characterized in that**:
   the device is a hand-held device and
   wherein the ultrasound element (2) is mounted to the magnetic element (3) so as to have a fixed spatial relationship to the magnetic element (3).

2. The device of claim 1, wherein the magnetic element (3) is configured such that a magnitude of magnetic force exerted on microbubbles varies along an axis extending through the magnetic element (3) and the magnetic force has a peak located a finite distance from the magnetic element (3) on the axis and within the target volume, and the ultrasound element (2) is configured such that a focal point of the acoustic field is located within the target volume.

3. The device of claim 3 wherein the ultrasound element (2) is configured such that an axis through the focal point of the acoustic field and the ultrasound element (2) is co-aligned with said axis extending through the magnetic element (3).

4. The device of any previous claim, wherein the ultrasound element (2) is mounted within a recess (31) in the magnetic element (3).

5. The device of claim 4, wherein a first part (32) of the magnetic element (3) is annular in shape and defines a circular recess.

6. The device of claim 4, wherein a first part (32) of the magnetic element (3) is circular in shape and is surrounded by an annular recess.

7. The device of any one of claims 5 or 6, wherein a second part (33) of the magnetic element (3) is detachably connected to the first part (32) of the magnetic element (3) and is arranged behind the first part (32) relative to the target volume.

8. The device of any one of claims 5 to 7, wherein the first part (32) of the magnetic element (3) is tapered towards the target volume.

9. The device of claim 7 or 9, wherein the second part (33) of the magnetic element (3) is tapered away from the target volume.

10. The device of any one of claims 5 to 9, wherein the first part (32) of the magnetic element (3) comprises a channel (4) therethrough for cooling the ultrasound element (2), the channel (4) being in communication with the recess (31) .

11. The device of any previous claim, wherein the magnetic element (3) is formed from one or more bodies of permanently magnetic material.

12. The device of claim 11, wherein the one or more bodies of magnetic material are shaped and/or mutually arranged so as to provide the magnetic field configured to retain magnetic microbubbles within the target volume.

13. The device of claim 11 or 12, wherein the magnetic element (3) comprises first and second bodies, the first and second bodies having the same magnetisation direction.

14. The device of claim 11, 12 or 13, wherein the magnetic material comprises NdFeB.

15. The device of any previous claim, wherein the ultrasound element (2) is connected to the magnetic element by a flexible material (23).

**Patentansprüche**

1. Vorrichtung zur Durchführung einer magnetischen Wirkstoffabzielung auf einen Patienten, wobei die Vorrichtung Folgendes umfasst:

ein magnetisches Element (3), das dafür konfiguriert ist, ein magnetisches Feld zu erzeugen, das dafür konfiguriert ist, magnetische Mikrobläschen in einem Zielvolumen zurückzuhalten; und
ein Ultraschallelement (2), das dafür konfiguriert ist, ein akustisches Feld bereitzustellen, das dafür konfiguriert ist, die magnetischen Mikrobläschen anzuregen, während die magnetischen Mikrobläschen durch das Magnetfeld innerhalb des Zielvolumens gehalten werden, **dadurch gekennzeichnet, dass**:

die Vorrichtung eine handgehaltene Vorrichtung ist und
wobei das Ultraschallelement (2) an dem magnetischen Element (3) so angebracht ist, dass es eine feste räumliche Beziehung zu dem magnetischen Element (3) hat.

2. Vorrichtung nach Anspruch 1, wobei das magnetische Element (3) dafür konfiguriert ist, eine Größe der auf die Mikrobläschen ausgeübten Magnetkraft entlang einer durch das magnetische Element (3) verlaufenden Achse zu variieren und die Magnetkraft eine Spitze aufweist, die sich in einem endlichen Abstand von dem Magnetelement (3) auf der Achse und innerhalb des Zielvolumens befindet, und das Ultraschallelement (2) dafür konfiguriert ist, dass sich ein Brennpunkt des akustischen Feldes innerhalb des Zielvolumens befindet.

3. Vorrichtung nach Anspruch 3, wobei das Ultraschallelement (2) dafür konfiguriert ist, eine Achse durch den Brennpunkt des akustischen Feldes und das Ultraschallelement (2) mit der Achse, die sich durch das magnetische Element (3) erstreckt, zu fluchten.

4. Vorrichtung nach einem der vorhergehenden Ansprüche, wobei das Ultraschallelement (2) in einer Ausnehmung (31) in dem magnetischen Element (3) angebracht ist.

**5.** Vorrichtung nach Anspruch 4, wobei ein erster Teil (32) des magnetischen Elements (3) ringförmig ist und eine kreisförmige Ausnehmung definiert.

**6.** Vorrichtung nach Anspruch 4, wobei ein erster Teil (32) des magnetischen Elements (3) kreisförmig ist und von einer ringförmigen Ausnehmung umgeben ist.

**7.** Vorrichtung nach einem der Ansprüche 5 oder 6, wobei ein zweiter Teil (33) des magnetischen Elements (3) mit dem ersten Teil (32) des magnetischen Elements (3) lösbar verbunden ist und in Bezug auf das Zielvolumen hinter dem ersten Teil (32) angeordnet ist.

**8.** Vorrichtung nach einem der Ansprüche 5 bis 7, wobei der erste Teil (32) des magnetischen Elements (3) in Richtung des Zielvolumens verjüngt ist.

**9.** Vorrichtung nach Anspruch 7 oder 9, wobei der zweite Teil (33) des magnetischen Elements (3) sich von dem Zielvolumen weg verjüngt.

**10.** Vorrichtung nach einem der Ansprüche 5 bis 9, wobei der erste Teil (32) des magnetischen Elements (3) einen Kanal (4) zur Kühlung des Ultraschallelements (2) aufweist, wobei der Kanal (4) mit der Ausnehmung (31) in Verbindung steht.

**11.** Vorrichtung nach einem der vorhergehenden Ansprüche, wobei das magnetische Element (3) aus einem oder mehreren Körpern aus permanentmagnetischem Material gebildet ist.

**12.** Vorrichtung nach Anspruch 11, wobei der eine oder die mehreren Körper aus magnetischem Material so geformt und/oder zueinander angeordnet sind, dass sie das Magnetfeld erzeugen, das so konfiguriert ist, dass magnetische Mikrobläschen in dem Zielvolumen zurückgehalten werden.

**13.** Vorrichtung nach Anspruch 11 oder 12, wobei das magnetische Element (3) einen ersten und einen zweiten Körper umfasst, wobei der erste und der zweite Körper die gleiche Magnetisierungsrichtung aufweisen.

**14.** Vorrichtung nach Anspruch 11, 12 oder 13, wobei das magnetische Material NdFeB umfasst.

**15.** Vorrichtung nach einem der vorhergehenden Ansprüche, wobei das Ultraschallelement (2) mit dem magnetischen Element durch ein flexibles Material (23) verbunden ist.

## Revendications

**1.** Dispositif pour effectuer un ciblage magnétique de médicament sur un patient, comprenant :

un élément magnétique (3) configuré pour fournir un champ magnétique configuré pour retenir des microbulles magnétiques dans un volume cible ; et
un élément à ultrasons (2) configuré pour fournir un champ acoustique configuré pour exciter les microbulles magnétiques tandis que les microbulles magnétiques sont retenues par le champ magnétique à l'intérieur du volume cible, **caractérisé en ce que** :

le dispositif est un dispositif portatif et
dans lequel l'élément à ultrasons (2) est monté sur l'élément magnétique (3) de manière à avoir une relation spatiale fixe avec l'élément magnétique (3).

**2.** Dispositif selon la revendication 1, dans lequel l'élément magnétique (3) est configuré de sorte qu'une amplitude de force magnétique exercée sur des microbulles varie le long d'un axe s'étendant à travers l'élément magnétique (3) et la force magnétique a un pic situé à une distance finie de l'élément magnétique (3) sur l'axe et à l'intérieur du volume cible, et l'élément à ultrasons (2) est configuré de sorte qu'un point focal du champ acoustique est situé à l'intérieur du volume cible.

**3.** Dispositif selon la revendication 3, dans lequel l'élément à ultrasons (2) est configuré de sorte qu'un axe passant par le point focal du champ acoustique et l'élément à ultrasons (2) est coaligné avec ledit axe s'étendant à travers l'élément magnétique (3).

4.  Dispositif selon l'une quelconque des revendications précédentes, dans lequel l'élément à ultrasons (2) est monté dans un évidement (31) de l'élément magnétique (3).

5.  Dispositif selon la revendication 4, dans lequel une première partie (32) de l'élément magnétique (3) est de forme annulaire et définit un évidement circulaire.

6.  Dispositif selon la revendication 4, dans lequel une première partie (32) de l'élément magnétique (3) est de forme circulaire et est entourée d'un évidement annulaire.

7.  Dispositif selon l'une quelconque des revendications 5 ou 6, dans lequel une deuxième partie (33) de l'élément magnétique (3) est reliée de manière amovible à la première partie (32) de l'élément magnétique (3) et est agencée derrière la première partie (32) par rapport au volume cible.

8.  Dispositif selon l'une quelconque des revendications 5 à 7, dans lequel la première partie (32) de l'élément magnétique (3) est effilée vers le volume cible.

9.  Dispositif selon la revendication 7 ou 9, dans lequel la deuxième partie (33) de l'élément magnétique (3) est effilée à l'opposé du volume cible.

10. Dispositif selon l'une quelconque des revendications 5 à 9, dans lequel la première partie (32) de l'élément magnétique (3) comprend un canal (4) traversant celle-ci pour refroidir l'élément à ultrasons (2), le canal (4) étant en communication avec l'évidement (31).

11. Dispositif selon l'une quelconque des revendications précédentes, dans lequel l'élément magnétique (3) est formé d'un ou plusieurs corps de matériau magnétique permanent.

12. Dispositif selon la revendication 11, dans lequel l'un ou plusieurs corps de matériau magnétique sont façonnés et/ou agencés mutuellement de manière à fournir le champ magnétique configuré pour retenir les microbulles magnétiques à l'intérieur du volume cible.

13. Dispositif selon la revendication 11 ou 12, dans lequel l'élément magnétique (3) comprend des premier et deuxième corps, les premier et deuxième corps ayant la même direction de magnétisation.

14. Dispositif selon la revendication 11, 12 ou 13, dans lequel le matériau magnétique comprend du NdFeB.

15. Dispositif selon l'une quelconque des revendications précédentes, dans lequel l'élément à ultrasons (2) est relié à l'élément magnétique par un matériau souple (23).

# Fig. 1(a)

# Fig. 1(b)

# Fig. 1(c)

# Fig. 1(d)

# Fig. 2

# Fig. 3(a)

# Fig. 3(b)

# Fig. 3(c)

# Fig. 3(d)

Fig. 4(a)

Fig. 4(b)

Fig. 4(c)

Fig. 4(d)

Fig. 5(a)

Fig. 5(b)

Fig. 6

Fig. 7

Fig. 8(a)

Fig. 8(b)

Fig. 9(a)

Fig. 9(b)

Fig. 9(c)

Fig. 9(d)

# Fig. 10(a)

# Fig. 10(b)

# Fig. 11

Build initial array based on optimization parameters → Calculate normalized force on particle at POI due to starting array → Start at element closest to POI → Test all possible magnetizations at element position

Set current array as new starting array ← Treat the next closest element to the POI

Does the total volume of magnetized elements exceed $V_{mag}$? — No — Retain magnetization that results in best normalized force

Has the process resulted in an improved array? — Yes — Have all elements been treated? — Yes (No)

Demagnetize element that contributes least to normalized force

Array optimized

EP 3 691 798 B1

Fig. 12(a)

Fig. 12(b)

Fig. 12(c)

Fig. 12(d)

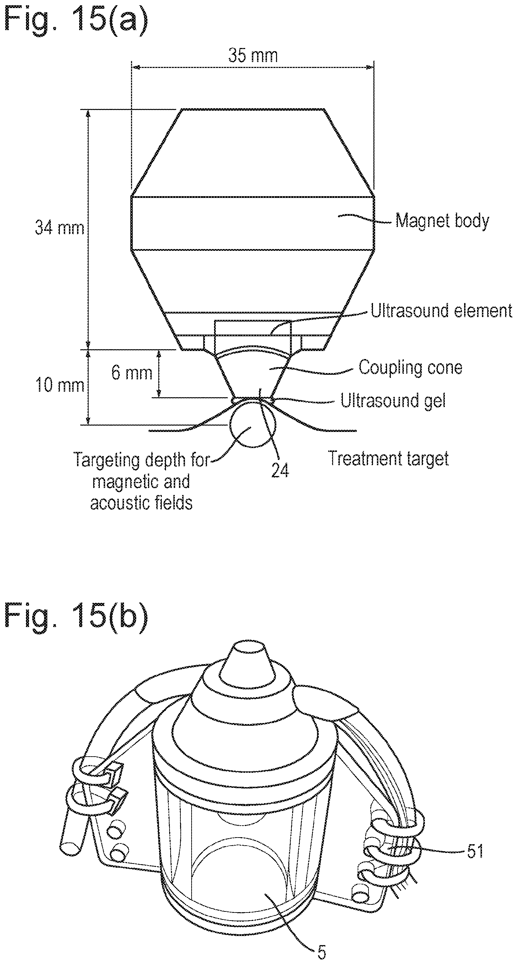

# Fig. 15(a)

35 mm

34 mm

Magnet body

Ultrasound element

Coupling cone

6 mm

10 mm

Ultrasound gel

Treatment target

Targeting depth for
magnetic and
acoustic fields

24

# Fig. 15(b)

51

5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011172486 A

**Non-patent literature cited in the description**

- **HALBACH K.** Design of permanent multipole magnets with oriented rare earth cobalt material. *Nuclear Instruments and Methods,* 1980, vol. 169, 1-10
- **STRIDE E. et al.** Halbach arrays consisting of cubic elements optimised for high field gradients in magnetic drug targeting applications. *Physics in Medicine and Biology,* 2015, vol. 60, 8303
- **DUCK F.** Physical Properties of Tissue: A Comprehensive Reference Book. Academic Press, 1990
- **STRIDE E et al.** Enhancement of Microbubble Mediated Gene Delivery by Simultaneous Exposure to Ultrasonic and Magnetic Fields. *Ultrasound in Medicine & Biology,* 2009, vol. 35, 861-868
- **SENNOGA C A et al.** On Sizing and Counting of Microbubbles Using Optical Microscopy. *Ultrasound in Medicine & Biology,* 2010, vol. 36, 2093-2096
- **STRIDE E. et al.** Understanding the dynamics of superparamagnetic particles under the influence of high field gradient arrays. *Physics in Medicine and Biology,* 2017, vol. 62, 2333